# EUROPÄISCHE PATENTANMELDUNG

(11) **EP 1 726 951 A1**
(43) Veröffentlichungstag der Anmeldung: **29.11.2006**
(21) Anmeldenummer: 06114222.0
(22) Anmeldetag: 19.05.2006
(51) Int. Cl.: G01N 33/487

(54) **Magazine zur Aufnahme von Testelementen**

(30) Priorität: 24.05.2005 EP 05011188
(71) Anmelder: F. Hoffmann-la Roche AG, 4070 Basle (CH); Roche Diagnostics GmbH, 68305 Mannheim (DE)
(72) Erfinder: Schulat, Jochen, 68167 Mannheim (DE); Mueller, Josef, 9533 Kirchberg SG (CH); Braendle, Hansjörg, 9244 Niederuzwil (CH)
(74) Vertreter: Dick, Alexander

(57) **Zusammenfassung**

Die Erfindung bezieht sich auf ein tragbares Analysegerät (10) mit einem von einem Gehäuse (12, 14) begrenzten Innenraum und einem Magazin (26, 78) zur Aufnahme von Testelementen (42). Es ist eine Fördereinrichtung (32, 34, 36) zum Fördern Testelemente (42) von einer ersten Position im Inneren des Magazins (26, 78) in eine zweite, zumindest teilweise außerhalb des Magazins (26, 78) und innerhalb des Gehäuses (12, 14) liegende Position vorgesehen, wobei das Fördern der Testelemente (42) in zwei Bewegungen erfolgt. Dabei werden die Testelemente (42) aus dem Magazin (26, 78), in dem die Testelemente (42) in einer Speicherposition (110, 114) enthalten sind, mittels einer Verdreheinrichtung (35, 36; 82) in einer Darreichungsposition (72, 100, 116) bewegt.

## Beschreibung

Die Erfindung bezieht sich auf ein Magazin zur Aufnahme von medizinischem Verbrauchsmaterial, wobei das Magazin insbesondere in einem tragbaren Analysesystem zur Analyse einer menschlichen Körperflüssigkeit eingesetzt werden kann.

### Stand der Technik

Aus EP 1 321 769 A1 ist ein Gerät bekannt, welches eine Spendeeinrichtung aufweist. Die Spendeeinrichtung umfasst ein Gehäuse mit einer Kammer. Eine Anzahl von Teststreifen wird in einer im Wesentlichen feuchtigkeitsdichten und luftdichten ersten Position gehalten. Es sind Einrichtungen vorgesehen zum Öffnen der Kammer und zum Bewegen eines der mehreren Teststreifen in translatorischer Richtung aus der ersten Position innerhalb der Kammer in zumindest eine teilweise außerhalb der Kammer liegende zweite Position. Das Öffnen der Kammer und das Herausbewegen des einen Teststreifens werden durch eine einzelne mechanische Bewegung erreicht. Ferner ist eine Analyseeinrichtung vorgesehen zur Analyse einer biologischen Flüssigkeit.

Aus WO 02/18940 A2 ist eine Testeinrichtung bekannt. Mittels der Testeinrichtung wird ein Fluid auf eine darin enthaltene Konzentration eines Analyten untersucht. Ein Gehäuse weist eine Öffnung auf und enthält einen Stapel von Sensoren. Ein Transportelement ist verdrehbar in der Öffnung des Gehäuses aufgenommen und hat eine Rotationsachse, welche in die Öffnung eingreift. Mittels einer Feder wird der Stapel gegen das Transportelement gedrückt. Ferner sind Dichtmittel vorgesehen, die eine feuchtigkeitsdichte Abdichtung zwischen dem Transportelement und den Sensoren bewirken, wenn sich das Transportelement in eine spezifische Drehposition bewegt. Eine Außenoberfläche des Transportelementes weist eine zurückspringende Ausnehmung auf, welche so gestaltet ist, dass ein einzelner Sensor vom Stapel aufgenommen werden kann. Eine Drehbewegung des Transportelementes mit einem in der zurückspringenden Ausnehmung aufgenommenen Sensor transportiert den Sensor an eine Position, in der der Sensor mit einem Messgerät verbunden werden kann und ein Tropfen des zu testenden Fluids aufnimmt.

Bei bisher eingesetzten Messgeräten zur Bestimmung beispielsweise des Glukosegehaltes von Blut, können einzelne als Testsreifen ausgebildete medizinische Verbrauchsmaterialien vom Benutzer eines derartigen Messgerätes per Hand in dieses eingeschoben werden. Die einzelnen Teststreifen und das Messgerät werden getrennt voneinander transportiert. Der Anzahl von Teststreifen, die der Anwender des Messgerätes mit sich führt, ist ein Code-Key beigelegt, der chargespezifische Informationen der Chemie enthält, die zur richtigen Bestimmung des Glukosegehaltes einer menschlichen Körperflüssigkeit wie z.B. Blut, notwendig sind und diese Informationen dem Messgerät übermittelt. Dieser Code-Key muss vor der Messung in das Messgerät eingeschoben werden. Um den Endanwendern die umständliche Handhabung von Teststreifen, Code-Key und Messgerät zu vereinfachen, gibt es Messgeräte, die ein Teststreifenmagazin aufweisen, welches in das Gerät eingelegt werden kann. Solche Geräte sind aus den eingangs zum Stand der Technik erwähnten EP 1 321 769 A1 und WO 02/18940 A2 bekannt. Gemäß diesen Lösungen können chargespezifische Informationen auf dem Magazin mitgeführt werden und durch das Messgerät automatisch ausgelesen werden.

Bei den aus dem Stand der Technik bekannten Lösungen ist der Umstand nachteilig, dass diese Lösungen relativ groß bauen, was die Messgeräte, in welche ein Magazin mit mehreren darin aufgenommenen Teststreifen eingeschoben wird, relativ voluminös macht. Dies jedoch ist durch den Endanwender höchst unerwünscht, da Messgeräte relativ unauffällig am Körper mitgeführt werden und etwa in Form eines Taschenrechners oder eines Mobiltelefons ausgelegt sein sollten, was das Handling, insbesondere das Mitführen solcher Messgeräte durch den Endanwender erleichtert. Ferner ist von Nachteil, dass die Messgeräte eine wenig benutzerfreundliche Formgebung aufweisen und sich die Darreichungsposition des Testelementes nicht in der optimalen Position befindet, was die Handhabung solcher Messgeräte erschwert.

### Darstellung der Erfmdung

Angesichts der vorstehend zitierten Nachteile der aus dem Stand der Technik bekannten Lösungen liegt der Erfindung die Aufgabe zugrunde, eine möglichst große Anzahl von Teststreifen feuchtigkeitsgeschützt in einem auswechselbaren Teststreifenmagazin in einem tragbaren Analysesystem unterzubringen.

Erfmdungsgemäß wird diese Aufgabe durch die Merkmale der unabhängigen Patentansprüche gelöst.

Der vorgeschlagenen Lösung folgend, wird ein Stapelmagazin vorgeschlagen, welches die Möglichkeit bietet, die darin enthaltenen Testelemente in einer größtmöglichen Packungsdichte zu magazinieren. Dadurch wird nicht nur die Möglichkeit geschaffen, das tragbare Analysegerät kleinbauend auszuführen, sondern auch eine größere Anzahl von Testelementen in dem auswechselbar konfigurierten Magazin unterzubringen. Am Magazin ist ein Code-Key integriert, der automatisch beim Einschieben des die Testelemente enthaltenden Magazins in das tragbare Analysesystem ausgelesen wird. Mittels des magazinintegrierten Code-Keys, werden chargenspezifische Informationen hinsichtlich der auf den Testelementen enthaltenen chemischen Substanzen übertragen, die zur richtigen Bestimmung des betreffenden Analyten z.B. des Glukosegehaltes von Blut erforderlich sind und an das tragbare Analysegerät übermittelt werden. Durch die Integration dieses Code-Keys am Magazin wird dem Patienten eine umständliche Handhabung mehrerer Komponenten, d.h. Testelement, Code-Key und Gerät vereinfacht, da der Code-Key und das die Anzahl von Testelementen aufnehmende Magazin ein Bauteil darstellen.

Das erfindungsgemäß vorgeschlagene z.B. in Stapelform konfigurierte Magazin bietet die Möglichkeit, sowohl einer automatischen als auch einer händischen Testelementbereitstellung. Wird insbesondere eine auf manuellem Wege bedienbare Form des Magazins gewählt, so lassen sich auch Antriebe sowie die für diese erforderlichen Energiequellen vermeiden, so dass das tragbare Analysegerät zusätzlich erheblich kleiner, bzw. robuster ausgelegt werden kann. Bisher aus dem Stand der Technik bekannte z.B. trommelförmig konfigurierte Systeme erfordern durch ihre rotatorische Weiterbewegung einen separaten, meistens elektrisch ausgelegten Antrieb.

Um den Eintritt von Luftfeuchtigkeit in das Innere des tragbaren Analysegerätes und in das Innere des stapelförmig ausgebildeten Magazins zu verhindern, werden aus einem elastischen Material wie z.B. Gummi gefertigte Dichtelemente eingesetzt. Diese werden beim Transport des Testelementes aus dem z.B. in Stapelform ausgebildeten Magazin innerhalb des tragbaren Analysegerätes bzw. Analysesystems geöffnet. Werden die Gummilippen durch das Testelement geöffnet, wird der Eintritt von Luftfeuchtigkeit in das Innere des in Stapelform konfigurierten Magazins durch das aus diesem austretende Testelement selbst bewirkt. Die Feuchtigkeit, die beim Aufdrücken der aus elastischem Material gefertigten Dichtelemente z.B. Dichtlippen, in den Innenraum des Magazins eindringen könnte, wird von einem innerhalb des z.B. in Stapelform ausbildbaren Magazins vorhandenen Trockenmittel z.B. Silica Gel absorbiert.

Gemäß einer ersten Ausführungsvariante des erfindungsgemäß vorgeschlagenen in Stapelform ausbildbaren Magazins können die Testelemente in schlitzförmig ausgebildeten Hohlräumen aufgenommen sein. Mittels eines manuell betätigten Stößels werden die einzelnen Testelemente aus den Hohlräumen ausgeschoben, die jeweils an der Eintrittsseite des Stößels in das z.B. in Stapelform ausgebildete Magazin und an der Austrittsseite des in Stapelform ausführbaren Magazins mittels elastischer Dichtelemente verschlossen sein können. Die Längsseiten des in Stapelform ausbildbaren Magazins können durch eine dünne Lackschicht oder eine Siegelfolie abgedichtet sein, die einen Eintritt von Luftfeuchtigkeit in die Hohlräume, in denen Testelemente aufgenommen sind, verhindert. Der Dichtlack oder die Siegelfolie werden dabei entweder vom Stößel auf der Eintrittsseite oder vom Testelement selbst durchstoßen.

Das z.B. in Stapelform ausbildbare Magazin umfasst eine zahnförmig ausgebildete Struktur, in welche eine Transportklinke eingreift. Mittels der Transportklinke wird das durch ein Vorspannelement beaufschlagte in Stapelform ausgebildete Magazin im Inneren des tragbaren Analysegerätes bewegt, so dass bei jedem Betätigungsvorgang durch den Patienten der Ausschub eines neuen, noch unbenutzten versiegelten Testelementes mittels des manuell betätigenden Stößels gewährleistet ist. Gemäß dieser Ausführungsvariante sind die einzelnen Testelemente innerhalb des Magazins in vertikaler Orientierung, d.h. hochkant aufgenommen. Der Stößel, der die Testelemente aus den Hohlräumen des Magazins ausschiebt, ist in einer dem Stößel eine Verdrehbewegung aufprägenden Führung geführt. Nachdem das Testelement aus dem Hohlraum innerhalb des Magazins ausgeschoben ist, wird dem Stößel und dem an der Stößelstirnseite aufgenommenen Testelement eine Verdrehbewegung aufgeprägt. Die Verdrehbewegung des Stößels mit dem daran aufgenommenen Testelement erfolgt erst, nachdem das Testelement den jeweiligen Hohlraum innerhalb des Magazins vollständig verlassen hat. So wird das Testelement von seiner Speicherposition, in der es eine Vertikallage annehmen kann, in eine horizontale Position überführt, d.h. das Testelement wird nach dem Ausschiebevorgang aus dem Magazin um z.B. 90° verdreht. In dieser um 90° verdrehten Position in Bezug auf die Lage des Testelementes im Magazin, tritt das Testelement aus dem tragbaren Analysegerät an einer Ausgabeöffnung aus. Durch die Verdrehung des Testelementes nach dem Ausschub aus dem Magazin kann erreicht werden, dass pro Magazin eine größere Anzahl von Testelementen im Magazin untergebracht werden kann, d.h. die Packungsdichte der Testelemente wird beträchtlich erhöht, im Vergleich zu einer horizontalen Anordnung der Testelemente im Magazin. Des Weiteren liegt das Testelement nach seiner beispielsweise um 90° erfolgenden Verdrehung in derselben Ebene wie das Display des tragbaren Analysegerätes bzw. des tragbaren Analysesystems. Dieser um 90° verdrehten Darreichungsposition des Testelementes kommt eine große Bedeutung zu. Dabei ist vor allem wichtig, dass der Patient bzw. der Benutzer eines Testgeräts sowohl die Stelle des Blutauftrags auf dem Testelement als auch das Display des Testgeräts mit einem Blick erkennt. Ferner ist auf diese Weise sichergestellt, dass das Testgerät sowohl für einen Rechtshänder als auch für einen Linkshänder ohne Probleme zu bedienen ist. In der Darreichungsposition liegt der Teststreifen parallel zum Display, d.h. in einer horizontalen Ebene, da der Bereich für den Blutauftrag in der Darreichungsposition auf der Oberseite des Testelementes liegt. Würde hingegen das Testelement sich in vertikaler Orientierung in der Darreichungsposition befinden, dann wäre der Benutzer des Testgeräts bzw. der Patient gezwungen, das Testgerät um 90° zu drehen, um die Blutauftragsstelle zu entdecken und wieder um 90° zurückzudrehen, um das Ergebnis auf dem Display des Testgeräts abzulesen. Je nach Drehrichtung und Geschicklichkeit des Benutzers würde in diesem Falle die Handhabung des Testgeräts erschwert.

Das gemäß der ersten Ausführungsvariante mit einer zahnförmig ausgebildeten Eingriffsstruktur für eine Transportklinke versehene Magazin wird mittels der Transportklinke innerhalb des tragbaren Analysegerätes transportiert. Mittels eines am Stößel zum Ausschub des Testelementes aufgenommenen Griffstücks wird die Transportklinke betätigt und schaltet dabei das Magazin derart weiter, dass dem über das Griffstück betätigbaren Stößel für den nächsten Ausschiebevorgang ein neuer unbenutzter nach außen luftdicht versiegeltes Testelement gegenüberliegt.

Gemäß eines weiteren Ausführungsbeispiels der erfindungsgemäß vorgeschlagenen Lösung kann ein Magazin, welches Testelemente enthält, in das tragbare Analysegerät bzw. Analysesystem eingeschoben werden. Innerhalb dieses Magazins sind die Testelemente schräg gestapelt, z.B. in einen Winkel von etwa 45° in Bezug auf die Bodenfläche dieses Magazins schräg gestapelt. Anstelle des Winkels von 45° könnte auch ein Winkel von 30° oder von 60° gewählt werden. Innerhalb des Magazins sind die in Schräglage angeordneten Testelemente durch eine federbeaufschlagte Fläche vorgespannt, welche bei Entnahme des in Schräglage aufgenommenen Testelementes aus dem Magazin, z.B. das nächste Testelement vorschiebt, so dass dieses bei manueller Betätigung eines Stößels durch einen Stempel oder dergleichen für den nächsten Entnahmevorgang bereit steht. Nach Einführen des Magazins, in welchem die Testelemente in Schräglage positioniert sind, kann das jeweils nächste Testelement aus dem Magazin entnommen werden. Vor Einlegen des Magazins in das tragbare Analysegerät bzw. das tragbare Analysesystem wird am Magazin eine Klappe geöffnet, welche eine Ausschiebeöffnung für das Testelement freigibt. Das an der Öffnungsstelle geöffnete Magazin wird nun seitlich in das tragbare Analysegerät bzw. das tragbare Analysesystem hineingeschoben. Der Ausschiebeöffnung des in Schräglage innerhalb des Magazins orientierten Testelementes gegenüberliegend, befmdet sich eine Verdreheinrichtung. Die Verdreheinrichtung umfasst z.B. zwei einander gegenüberliegend federnd gelagerte Anschlagflächen. Jede dieser federnd gelagerten Anschlagflächen umfasst eine Einführschräge. Aufgrund der federnden Lagerung der beiden Anschlagflächen in Bezug aufeinander, ist eine variable Spaltbreite zwischen einem engen Spalt zwischen den Anschlagflächen und einem weiten Spalt möglich. Sobald das die Verdreheinrichtung erreichende vorlaufende Ende des Testelementes in 45° Schräglage orientiert, die Einlaufschrägen der beiden federnd aneinander gestellten Anlageflächen erreicht, weitet sich bei weiterem Vorschub des Stößels während der Ausschubbewegung des Testelementes aus dem Magazin der Spalt zwischen den beiden einander gegenüberliegenden positionierten Anschlagflächen auf. Ist das Testelement aus dem Magazin durch den Stößel vollständig ausgeschoben, wird das Testelement von seiner 45° Lage, bewirkt durch die an den relativ zueinander beweglichen Anschlägen vorgesehenen Federn, in eine Horizontallage überführt, d.h. um etwa 45° gedreht.

Analog zum ersten Ausführungsbeispiel liegt das aus dem Magazin ausgeschobene Testelement nunmehr in einer Ebene, d.h. einer sich horizontal erstreckenden Ebene parallel zum an dem tragbaren Analysegerät bzw. Analysesystem angeordneten Display. Aufgrund dieser Darreichungsposition, in welcher das Testelement parallel zu dem am tragbaren Analysegerät bzw. Analysesystem vorhandenen Display liegt, sind sowohl der für z.B. einen Blutauftrag bestimmte Bereich des Testelementes als auch das Display des tragbaren Analysegeräts bzw. Analysesystems auf einen Blick erkennbar, ohne dass das tragbare Analysegerät bzw. Analysesystem durch den Patienten bzw. den Benutzer zu verdrehen ist. Dadurch lässt sich das tragbare Analysegerät bzw. das tragbare Analysesystem sowohl durch einen Rechtshänder als auch durch einen Linkshänder mit hohem Bedienungskomfort handhaben. Ein Verdrehen zur Erkennung des Blutauftragsbereichs des Testelementes und ein Rückdrehen zur Ablesung des Messergebnisses im Display des tragbaren Analysegeräts bzw. Analysesystems können nunmehr entfallen.

Mit der erfindungsgemäß vorgeschlagenen Lösung ist es möglich, einerseits die Packungsdichte von Testelementen innerhalb eines Magazins zu optimieren und andererseits zu gewährleisten, dass das jeweils zu benutzende Testelement, aus dem tragbaren Analysegerät bzw. dem tragbaren Analysesystem für den Benutzer in einer eine optimale Handhabung gewährleistenden Orientierung austritt.

Dies wird durch die den vorstehend skizzierten Ausführungsbeispielen gemeinsame Verdrehung des Testelementes innerhalb des tragbaren Analysegerätes bzw. des tragbaren Analysesystems gewährleistet. Die Verdrehung des Testelementes, sei es um 90° oder sei es um 45° innerhalb des Analysegerätes wird durch eine manuelle Betätigung desselben erreicht, die dem als Auswerfer fungierenden Stößel sowohl eine Hubbewegung als auch eine sich and die Hubbewegung anschließende Verdrehbewegung aufprägt. Dies geschieht bei nur einem Betätigungsvorgang durch den Patienten bzw. den Benutzer und bringt das Testelement in eine für die Anwendung optimale Position.

### Zeichnung

Anhand der Zeichnung wird die Erfmdung nachfolgend eingehender beschrieben.

### Es zeigt:

Figur 1 eine Explosionsdarstellung eines tragbaren Analysegerätes in einer ersten Ausführungsvariante mit getrennt voneinander dargestellter Ober- und Unterschale samt Einbauten,
Figur 2 das tragbare Analysegerät gemäß Figur 1 in zusammengefügtem Zustand mit in Einschiebrichtung einzuschiebendem Magazin,
Figur 3 die Komponenten einer Ausschubvorrichtung für die Testelemente sowie eine Transportklinke zum Vorschub des Magazins,
Figuren 4.1, 4.2, 4.3, 4.4 und 4.5 den Ausschub und den sich an den Ausschub anschließenden Verdrehvorgang des Testelementes von einer Vertikallage in eine Horizontallage,
Figur 5.1 und 5.2 den Vorschub des Magazins bei Vorwärtshub und Rückwärtshub der Transportklinke,
Figur 6 ein weiteres Ausführungsbeispiel eines tragbaren Analysegerätes bzw. Analysesystems in Explosionsdarstellung,
Figur 7 das tragbare Analysegerät bzw. Analysesystem im zusammengefügten Zustand,
Figur 7.1 eine Detaildarstellung des in das tragbare Analysegerät gemäß Figur 7 einschiebbaren Magazin mit seitlicher Entnahmeklappe,
Figur 8.1 das tragbare Analysegerät gemäß Figur 7 mit nicht betätigter Ausschubvorrichtung,
Figur 8.2 das durch Betätigung eines Druckstempels aus dem tragbaren Analysegerät gemäß Darstellung in Figur 7 ausgeschobene Testelement,
Figur 9.1 dass die Testelemente in Schräglage enthaltende Magazin mit einer Verdreheinrichtung,
Figur 9.2 die Verdreheinrichtung mit aus dem Magazin herausgeschobenen Testelement,
Figur 9.3 eine Innenansicht des Magazins gemäß Darstellung in Figur 7.1,
Figur 9.4 ein in eine horizontale Position verdrehtes Testelement, gehalten durch die Verdreheinrichtung und
Figuren 9.5 bis 9.8 mehrere Stadien des Verdrehvorganges des Testelementes innerhalb der Verdreheinrichtung des tragbaren Analysegerätes gemäß Figur 6, und
Figuren 10.1 und 10.2 ein klappenförmiges Abdichtelement zur Langzeitabdichtung eines stationär im Gehäuse angeordneten Magazins.

### Ausführungsvarianten:

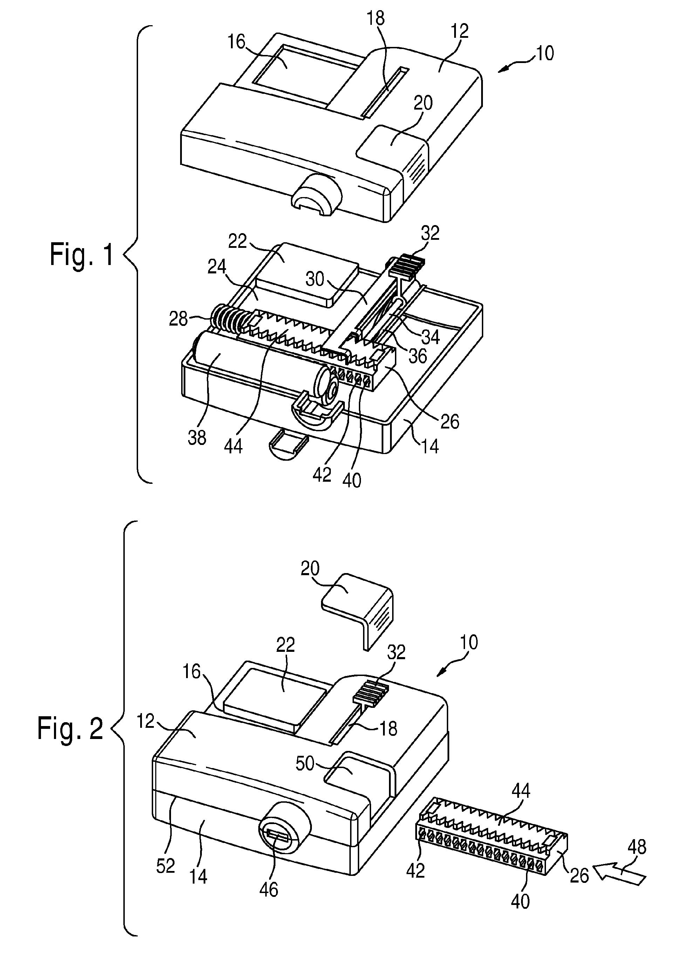

Figur 1 ist ein erstes Ausführungsbeispiel eines tragbaren Analysegerätes mit einem Testelemente enthaltenden Magazin in Explosionsdarstellung zu entnehmen.

Nachfolgend werden unter Messgeräten oder Analysegeräten portable Geräte verstanden, die ein Benutzer am Körper stets bei sich führen kann. Derartige transportable Messgeräte oder Analysegeräte enthalten einen Langzeitenergiespeicher, der eine im portablen Messgerät oder in dem portablen Analysegerät aufgenommene Auswerteelektronik mit Energie versorgt. Die Auswertung von Testelementen innerhalb des portablen Mess- oder Analysegerätes kann auf elektrochemischem Wege oder auch auf optischem Wege erfolgen. Bei einer optischen Auswerteelektronik wird ein Testelement, wie z. B. ein Teststreifen, mittels einer Anzahl von Strahlen abgetastet, während bei einer elektrochemischen Auswertung eines Testelementes auf einen in einer Körperflüssigkeit enthaltenen Analyten das Testelement eine bestimmte Testchemie aufweist.

Im Rahmen einer elektrochemischen Auswertung von Testelementen weisen diese vorzugsweise flach oder streifenförmig ausgebildeten Testelemente eine Grundfolie und eine Trägerfolie mit einer Reagenzschicht auf. Innerhalb der Trägerfolie verlaufen Leiterbahnen zum Anschluss von Elektroden. Oberhalb der Leiterbahnen kann die bereits erwähnte Reagenzschicht, welche die Testchemie enthält, aufgenommen sein. Zwischen der Reagenzschicht und einer Distanzfolie, die die Reagenzschicht in einzelnen Bereichen überdeckt, können Messkammern bzw. Messkapillarräume ausgebildet sein. Die elektrochemischen Messkammern bzw. Messkapillarräume können von einer hydrophilen Schicht überdeckt sein, die Ihrerseits über eine Deckelfolie abgedeckt ist. Zur Entlüftung der elektrochemischen Messzellen können sowohl die Deckelfolie als auch die unter dieser angeordnete hydrophile Schicht von einer Entlüftung durchzogen sein.

Die von der Distanzfolie einerseits sowie von der Reagenzschicht und der hydrophoben Schicht andererseits begrenzte elektrochemische Messzelle nimmt z. B. einander gegenüberliegend angeordnete Elektroden auf. Die Elektroden umfassen eine Gegenelektrode CE und eine weitere Elektrode WE. Diese können z. B. kammförmig ineinander greifen. Darüber hinaus können elektrochemischen Messzellen den Füllstand erfassende Elektroden FSE zugeordnet sein, wobei einer einzelnen elektrochemischen Messzelle jeweils immer ein Paar von deren Füllstand erfassenden Elektroden FSE zugeordnet sein kann.

Die Testelemente können hingegen auch als elektrochemische Kapillarsensoren ausgebildet sein. Ein solcher Kapillarsensorträger umfasst eine steifere Grundfolie mit einer leitfähigen Struktur, Elektronenflächen, Leiterbahnen und Kontakte. Über die steifer ausgebildete Grundfolie wird über eine Fließerbeschichtung im Bereich der Elektroden ein Reagenzfilm mit den für die gewünschte Messreaktion notwendigen Reagenzien aufgebracht. Darauf lässt sich eine gestanzte Distanzfolie aufbringen, so z. B. aufkleben. Diese wiederum bildet auf der einen Seite des Kapillarsensorträgers eine zu dieser Seite offene Kapillare sowie eine elektrochemische Messzelle über den Elektrodenflächen und gleichzeitig auf der anderen Seite an den Enden der Leiterbahn Kontaktflächen, an denen eine elektrische Kontaktierung vorgenommen werden kann. Auf die aufgebrachte, so z. B. aufgeklebte Reagenzfolie kann abschließend eine Deckelfolie aufgeklebt sein, die die Kapillaren nach oben abschließt und am inneren Ende der Kapillare ein Entlüftungsloch bildet.

Die Auswertung der einzelnen Testelemente, sei es auf optischem, sei es auf elektrochemischem Wege, erfolgt vorzugsweise innerhalb des Messgerätes bzw. des Analysegerätes. Die Auswertung kann auch bei teilweise in den Innenraum des Mess- bzw. Analysegerätes eingezogenem auszuwertenden, die menschliche Körperflüssigkeit enthaltenden Testelement vorgenommen werden.

Ein tragbares Analysegerät 10 umfasst eine Oberschale 12 sowie eine Unterschale 14. In der Oberschale 12 ist eine Öffnung 16 ausgebildet, durch welche im zusammengefügten Zustand des tragbaren Analysegerätes 10 ein Display 22 ablesbar ist. Ferner befindet sich in der Oberschale 12 des tragbaren Analysegerätes 10 gemäß Figur 1 eine schlitzförmig sich erstreckende Öffnung 18. In die Oberschale 12 des tragbaren Analysegerätes 10 ist darüber hinaus eine entfernbare Verschlussklappe 20 integriert.

In der Unterschale 14 des tragbaren Analysegerätes 10 befindet sich das Display 22, welches auf einer nicht näher dargestellten nur angedeuteten Platine 24 aufgenommen ist. In der Unterschale 14 befindet sich darüber hinaus auch ein Magazin 26 in Form eines Stapelmagazins, welches durch eine Vorspannfeder 28 beaufschlagt ist. Das Magazin 26 wird mittels einer Transportklinke 30 innerhalb des tragbaren Analysegerätes 10 bewegt. Das Magazin 26 ist mit einer Anzahl von Testelementen 42 bestückt. Das Magazin 26 enthält ein Trockenmittel wie zum Beispiel Silica Gel und ist nach außen hin durch aufgebrachte Folien oder durch eine Siegelschicht luftdicht verschlossen, so dass der Eintritt von Luftfeuchtigkeit in das Innere des Magazins 26 ausgeschlossen ist. Die das Innere des Magazins 26 abdichtende Dichtelemente, seien es dünne seitlich aufgebrachte Folien oder sei es ein Siegellack oder dergleichen sind so beschaffen, dass sie von einer Vorschubeinrichtung, wie zum Beispiel einem Stößel 34 durchstoßen werden können.

Die Transportklinke 30 erstreckt sich parallel zu einem mittels eines Griffstücks 32 betätigbaren Stößels 34. Der Stößel 34 seinerseits ist von einer Führung 36 umschlossen, welche dem Stößel 34 nach Durchfahren des Magazins 26 eine Verdrehbewegung aufträgt. In der Unterschale 14 des tragbaren Analysegerätes 10 sind darüber hinaus auch Energiespeicher 38 aufgenommen, welche das tragbare Analysegerät 10 mit elektrischer Energie versorgen.

Das in vorteilhafter Weise als Stapelmagazin ausgebildete Magazin 26 umfasst mehrere im Wesentlichen in vertikaler Orientierung angeordnete Aufnahmeräume 40 für Testelemente 42. An der Oberseite des Magazins 26 befindet sich eine Eingriffsstruktur 44, in welche an der Transportklinke 30 ausgebildete Mitnehmerzähne eingreifen. Dies wird nachfolgend noch eingehender beschrieben.

Unter Testelement 42 wird im Folgenden ein solches verstanden, welches Reagenzien enthält, mit welchen eine menschliche Körperflüssigkeit auf einen Analyten untersucht werden kann. Bei der menschlichen Körperflüssigkeit kann es sich zum Beispiel um Blut, sei es Vollblut sei es verdünntes Blut oder um andere Körperflüssigkeiten handeln. Das Testelement 42 kann darüber hinaus eine Stech-Funktionalität aufweisen, verwirklicht zum Beispiel durch eine in das Testelement 42 integrierte Lanzette sowie einen Spender für Lanzetten. Bei dem Testelement 42 kann es sich auch um ein integriertes Testelement handeln, bei dem eine Auswerteschaltung ganz oder teilweise auf dem Testelement integriert ist. Die Auswerteschaltung kann z.B. eine organische Elektronik unter Verwendung von OFET's sein. Integrierte Testelemente können darüber hinaus eine Optik enthalten, ferner Anregungslichtquellen wie z.B. OLED's als Lichtquellen. Ferner lässt sich in integrierte Testelemente eine Energiequelle in Form eines integrierten SuperCAP's integrieren. Integrierte Testelemente zeichnen sich durch eine hohe Anzahl integrierter Funktionen aus.

Während die Testelemente 42 auch eine Stechfunktionalität als integrierte Funktion in Form einer integrierten Lanzette aufweisen können, können die Stechhilfen auch separat bevorratet sein. Die als Lanzetten ausgebildeten Stechhilfen können z.B. in einem trommelförmig ausgebildeten Magazin angeordnet sein und unabhängig von den Testelementen im Messgerät aufgenommen sein. Hinsichtlich der Stechhilfen in Form von Lanzetten kann für jede Applikation, d.h. für jeden Gebrauchsvorgang, eine neue Stechhilfe zur Verfügung gestellt werden; es sind jedoch auch Applikationen eines tragbaren Analysegerätes denkbar, bei denen ein und dieselbe Stechhilfe mehrfach benutzt werden kann.

Figur 2 zeigt das tragbare Analysegerät gemäß des in Figur 1 dargestellten Ausführungsbeispieles in montiertem Zustand. Aus der Darstellung gemäß Figur 2 geht hervor, dass das Display 22 die Öffnung 16 der Oberschale 12 durchsetzt. Das Griffstück 32 zur Betätigung des Stößels 34 ist innerhalb der schlitzförmig ausgeführten Öffnung 18 verschiebbar. Eine Einschuböffnung 50 ist durch Entfernen der Verschlussklappe 20 freigelegt, so dass das die Testelemente 42 enthaltende Magazin 26 in Einschubrichtung 48 in das tragbare Analysegerät 10 einschiebbar ist. Eine Ausgabeöffnung 46 für die Testelemente 42 ist durch halbzylinderförmige Bereiche, die jeweils an der Oberschale 12 bzw. an der Unterschale 14 angespritzt sein können, begrenzt. Das noch unbenutzte Magazin 26 weist an seinen Längsseiten in Bezug auf die Einschubrichtung 48 gesehen jeweils Abdichtungen auf, um ein Eindringen von Luftfeuchtigkeit in die Aufnahmeräume 40, die im Wesentlichen vertikal orientiert sind, und in denen die Testelemente 42 enthalten sind, zu vermeiden.

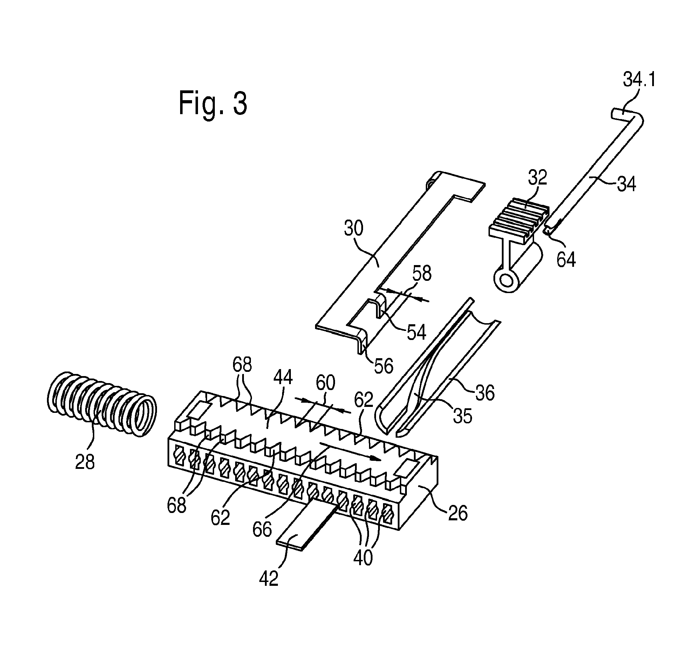

Figur 3 sind die Komponenten einer Ausschubmimik sowie einer Transportmimik für das Magazin zu entnehmen.

Der Stößel 34 weist ein abgewinkeltes Ende und an seiner dem Magazin 26 zuweisenden Seite einen Aufnahmeschlitz 64 auf. Auf dem Stößel 34 ist das schieberförmige Griffstück 32 mittels eines hülsenförmigen Ansatzes aufgenommen, so dass der Stößel 34 bei manueller Betätigung des schieberförmigen Griffstückes 32 bewegbar ist. Der Stößel 34 mit dem daran montierten schieberförmigen Griffstück 32 ist in einer in die Unterschale 14 des tragbaren Analysegerätes 10 eingelassenen Führung 36 bewegbar. An der Innenseite der Führung 36 befindet sich ein Kurvenabschnitt 35, welcher dem Stößel 34 eine Verdrehbewegung nach Durchfahren eines Hohlraums 40 des Magazins 26 und dem Ausschieben der Testelemente 42 aus diesem, aufprägt. Die zum Vorschub des Magazins 26, bevorzugt ein Stapelmagazin, in Vorschubrichtung 66 dienende Transportklinke 30 weist einen ersten Mitnehmerzahn 54 sowie einen zweiten Mitnehmerzahn 56 auf. Der erste Mitnehmerzahn 54 und der zweite Mitnehmerzahn 56 sind in einem Versatz 58 zu einander an der Transportklinke 30 ausgebildet. Die Mitnehmerzähne 54, 56 greifen in die Eingriffsstruktur 44 ein, die an der Oberseite des Magazins 26, das als Stapelmagazin ausgebildet sein kann, ausgeführt ist. In der Darstellung gemäß Figur 3 ist die Eingriffsstruktur 44 als eine Vielzahl von Zähnen ausgebildet, die in einer Teilung 60 in Bezug aufeinander angeordnet sind. Die Zähne der Eingriffsstruktur 44 sind durch Einlaufschrägen 62 bzw. Anschlagflächen 68 definiert und an beiden Längsseiten der Eingriffsstruktur 44 ausgebildet. Wie aus der Darstellung gemäß Figur 3 zudem hervorgeht, sind die einzelnen Aufnahmeräume 40 des Magazins 26 im Wesentlichen vertikal orientiert und weisen eine etwa mittig liegende Erweiterung auf, die eine Passage des Stößels 34 bei dessen Einfahren in den Aufnahmeraum 40 des Magazins 26 ermöglicht. Sobald der Stößel 34 das Magazin 26 senkrecht zu dessen Vorschubrichtung 66 durchfahren hat, wird das aus den Aufnahmeräumen 40 jeweils ausgeschobene Testelement 42 in die in Figur 3 dargestellte horizontale Position verdreht, was durch den Kurvenabschnitt 35 an der Innenseite der Führung 36 bewirkt wird.

Wie der Darstellung gemäß Figur 3 entnehmbar ist, umfasst eine Verdreheinrichtung für die Testelemente 42 den Stößel 34 mit einem abgewinkelt ausgebildeten Ende 34.1, sowie die Führung 36, in welcher der Stößel 34 geführt ist. Der innerhalb der Führung 36 angeordnete Kurvenabschnitt 35 prägt den Stößel 34 nach Ausschub des Testelementes 42 aus dem Magazin 26 die Verdrehung in einer Darreichungsposition auf. Unter Verdreheinrichtung wird im Folgenden eine Einrichtung verstanden, welche die Testelemente 42 von ihrer Hochkantlage, in welcher sie innerhalb des Magazins 26 gespeichert sind, in eine relativ zu der Hochkantlage verdrehte Lage bringen.

Der Darstellung gemäß Figur 3 sind darüber hinaus die wesentlichen Komponenten der Fördereinrichtung des Testelementes 42 zu entnehmen, mit welcher die Testelemente 42 aus dem Magazin 26 gefördert werden. Dabei handelt es sich um den Stößel 34 und das mit diesem gekoppelte Griffstück 32. Unter dem Terminus "Fördereinrichtung" werden solche Einrichtungen verstanden, mit welchen ein Testelement 42 aus dem Magazin 26 herausgeschoben werden kann, wobei die Fördereinrichtung sowohl manuell bedienbar ausgebildet sein kann oder auch automatisch arbeiten kann.

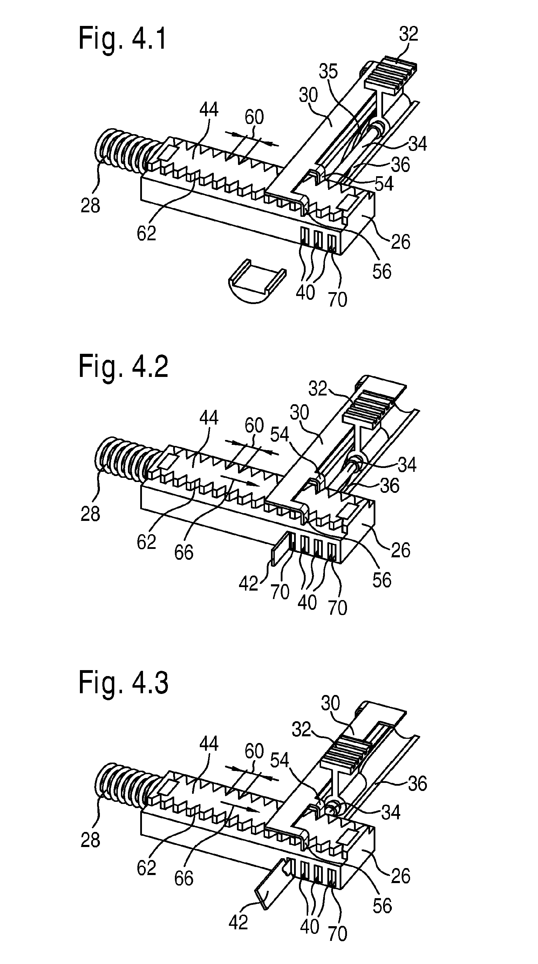

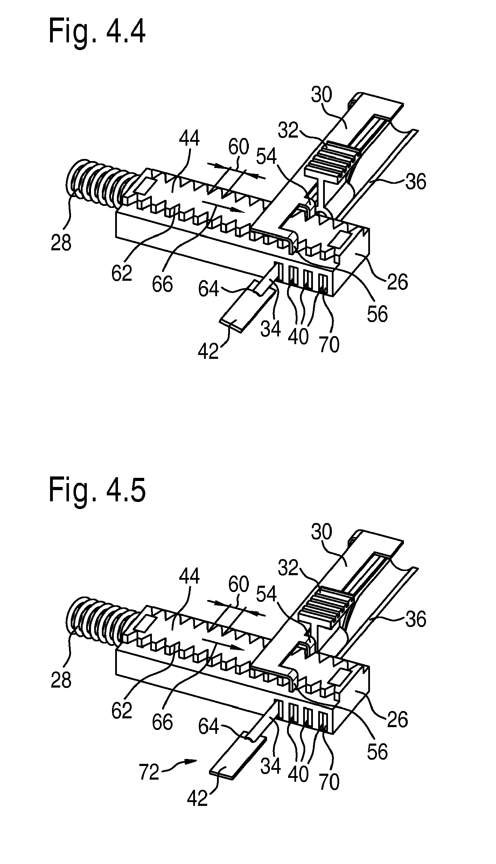

Aus der Darstellung gemäß Figur 4.1 geht die Ausschiebemimik für die Testelemente sowie die Transportmimik für das Magazin detaillierter hervor.

In der Darstellung gemäß Figur 4.1 ist das die Testelemente 42 enthaltende Magazin 26 durch die Transportklinke 30 in eine definierte Position innerhalb des tragbaren Analysegerätes 10 transportiert und dort arretiert. Dazu greift der erste Mitnehmerzahn 54 auf der diesem zuweisenden Seite der Eingriffsstruktur 44 ein, die in der Teilung 60 ausgebildet ist. Damit ist eine Verschiebung des Magazins 26, welches bevorzugt als Stapelmagazin ausgeführt ist, innerhalb des tragbaren Analysegerätes 10 nicht möglich. Das in der schlitzförmigen Öffnung 18 geführte schieberförmige Griffstück 32 schiebt bei manueller Betätigung den Stößel 34 in den dessen Stirnseite gegenüberliegenden Aufnahmeraum 40 des Magazins 26 ein und schiebt das in diesem enthaltene Testelement 42 (vergleiche Darstellung gemäß Figur 2) aus dem Aufnahmeraum 40 aus. Während der Vorschubbewegung des Stößels 34 ist der an der Innenseite der Führung 36 ausgeführte Kurvenabschnitt 35 ohne Wirkung. Während der Vorschubbewegung des Stößels 34, bei manueller Betätigung des schieberförmigen Griffstückes 32, erfolgt keine Verdrehung des Stößels 34.

In der in Figur 4.1 dargestellten Position des Magazins 26 ist dieses aufgrund des Eingriffes des ersten Mitnehmerzahns 54 in die Eingriffsstruktur 44 gegenüber der Wirkung der Vorspannfeder 28 arretiert. Bezugszeichen 70 bezeichnet ein aus elastischem Material gefertigtes, z.B. lippenförmig ausgebildetes Abdichtelement, welches im unbenutzten Zustand des Magazins 26 jeweils sowohl auf der dem Stößel 34 zuweisenden Längsseite als auch auf der der Ausgabeöffnung 46 (vergleiche Figur 2) zuweisenden Seite des Magazins 26 ausgebildet ist. Anstelle der in Figur 4.1 dargestellten lippenförmig ausgebildeten Abdichtelemente 70 können die Längsseiten des Magazins 26 auch mit einer Abdichtfolie oder auch mit einem Abdichtlack überzogen sein, welcher das Eindringen von Luftfeuchtigkeit in die Aufnahmeräume 40 des Magazins 26 und demzufolge eine Kontamination des darin enthaltenen Testelementes 42 mit Feuchtigkeit verhindert. Darüber hinaus ist im Innenraum des stapelförmigen Magazins 26 ein figürlich nicht dargestelltes Trockenmittel wie z.B. Silica Gel aufgenommen.

Der Darstellung gemäß Figur 4.2 ist entnehmbar, dass das schieberförmig ausgebildete Griffstück 32 und demzufolge der mit diesem verbundene Stößel 34 das Testelement 42 teilweise aus dessen Aufnahmeraum 40 im Magazin 26 bewegt hat. Aufgrund der Orientierung der Aufnahmeräume 40 im Magazin 26 befindet sich das Testelement 42 in diesem Zustand in einer im Wesentlichen vertikalen Position, d.h. das Testelement 42 wird hochkant aus dem Aufnahmeraum 40 ausgeschoben.

Unter Hochkantlage wird nachfolgend verstanden, dass die Testelemente 42 sich in einer im Wesentlichen vertikalen Position aufliegend auf einer ihrer Längsseiten befinden. Die Testelemente 42 sind bevorzugt streifenförmig ausgebildet, so dass durch eine derartige durch eine Hochkantlage charakterisierte Position der Testelemente 42 innerhalb des Magazins 26 eine sehr hohe Packungsdichte erreicht werden kann. Unter dem Begriff Hochkantlage wird nachfolgend sowohl eine senkrechte Lage der Testelemente 42 in Bezug auf eine Auflagefläche des Magazins 26 verstanden, als auch eine um einen Winkel geneigte Orientierung der Testelemente 42 auf einer Bezugsfläche des Magazins 26.

Während der Ausschubbewegung des Testelementes 42 aus dem Aufnahmeraum 40 des Magazins 26 liegen die beidseits der Austrittsöffnung des Aufnahmeraumes 40 angeordneten lippenförmigen Dichtelemente 70 an den Flächen des Testelementes 42 an und unterbinden somit das Eindringen von Luftfeuchtigkeit in das Innere des Magazins 26. Der zwangsläufig während der Ausschubbewegung des Testelementes 42 erfolgende Luftfeuchtigkeitseintritt in den Innenraum des Magazins 26 wird von dem erwähnten, im Innenraum des Magazins 26 aufgenommenen Vorrat eines Trockenmittels, wie z.B. Silica Gel absorbiert. Auch in der Darstellung gemäß Figur 4.2 ist das Magazin 26 durch Eingriff des ersten Mitnehmerzahns 54 in die Eingriffsstruktur 44 in seiner Position arretiert und ist in der jeweiligen Position des Magazins 26 im Inneren des tragbaren Analysegerätes 10 durch die komprimierte Vorspannfeder 28 beaufschlagt. Während der erste Mitnehmerzahn 54 in einen durch zwei Einlaufschrägen 62 begrenzten Zwischenraum der Eingriffsstruktur 44 gestellt ist, liegt der zweite Mitnehmerzahn 56 an einer Anschlagfläche 68 auf der gegenüberliegenden Seite der Eingriffsstruktur 44 an. Eine Bewegung des Magazins 26 in Vorschubrichtung 66 ist somit unterbunden.

Aus Figur 4.3 geht hervor, dass das von der als Schlitzung 64 beschaffenen Aufnahmeöffnung ergriffene Testelement 42 nach Ausschub aus seinem entsprechendem Aufnahmeraum 40 von seiner vertikalen Position ausgehend, verdreht ist. Es nimmt seine Darreichungsposition 72 ein, in welcher es in Bezug auf seine Speicherposition im Magazin 26 um 90° verdreht ist und damit (vgl. Darstellung gemäß Figur 2) parallel zum an der Oberschale 12 des tragbaren Analysegeräts 10 vorgesehenen Display 22 liegt. Der Stößel 34 umfasst ein abgewinkeltes Ende 34.1 (vgl. Darstellung gemäß Figur 3), welches beim Betätigen des Griffstücks 32 und dem Verfahren des Stößels 34 in der Führung 36 auf den an die Innenwand der Führung ausgebildeten Kurvenabschnitt 35 aufläuft. Dadurch wird dem Stößel 34 eine Verdrehbewegung aufgeprägt, die erst dann beginnt, sobald das Testelement 42 vollständig aus dem Hohlraum 40 des Magazins 26 ausgeschoben ist. Um die Vorschubbewegung des Stößels 34 innerhalb der Führung 36 mit darin ausgebildetem Kurvenabschnitt 35 zu gewährleisten, ist das Griffstück 32 über einen Anschlag an der Außenumfangfläche des Stößels 34 oder einen ringförmigen Ansatz oder dergleichen in Längsrichtung auf dem Stößel 34 gesichert, wobei der am unteren Ende des Griffstücks 32 ausgebildete, den Stößel 34 umgebende Hülsenabschnitt eine Verdrehbewegung zwischen dem Griffstück 32 und dem Stößel 34 zulässt. Diese Relativbewegung in Umfangsrichtung des Stößels 34 bewirkt die Verdrehung des Stößels 34 samt daran aufgenommenem Testelement 42, sobald das abgewinkelte Ende des Stößels 34 auf den Kurvenabschnitt 35 aufläuft, was erst der Fall ist, nachdem das Testelement 42 vollständig aus dem Hohlraum 40 des Magazins 26 ausgeschoben ist. Dadurch gelangt das Testelement 42 in die in Figur 4.5 dargestellte Darreichungsposition 72.

Unter Darreichungsposition wird die Position des Testelementes 42 verstanden, in welcher das Testelement 42 aus dem Mess- bzw. Analysegerät 10 austritt und dem Benutzer zur Anwendung dargeboten wird. Die Darreichungsposition 72 ist dadurch charakterisiert, dass das Testelement 42 in seiner Darreichungsposition 72 im Wesentlichen parallel oder mit einer geringen Neigung oder Verkippung zu dem an der Oberseite des Mess- bzw. Analysegerätes 10 enthaltenen Displays 22 positioniert ist. Der Anwender kann in der Darreichungsposition 72 sowohl das Auftragen einer Körperflüssigkeit auf das Testelement 42 ausführen als auch das Display 22 des Mess- bzw. Analysegerätes 10 ablesen, ohne das Mess- oder Analysegerät 10 verdrehen zu müssen. Die Darreichungsposition 72 vereinfacht somit die Handhabung eines Mess- bzw. Analysegerätes 10 erheblich, da ein Verdrehen desselben nunmehr entfallen kann, da die Darreichungsposition 72 in einer für den Anwender optimalen Position liegt.

Auch in der in Figur 4.3 dargestellten Position des Magazins 26 ist dieses durch den ersten Mitnehmerzahn 54 in seiner Position innerhalb der Unterschale 14 des tragbaren Analysegerätes 10 arretiert. Dadurch wird einerseits ein Verkanten des in den entsprechenden Aufnahmeraum 40 eingeschobenen Stößels 34 vermieden, andererseits die Erstreckung des Aufnahmeraumes 40 im Magazin 26 als Führungsfläche für den Stößel 34 genutzt. In der in Figur 4.3 dargestellten Position des Testelementes 42 ist das lippenförmig ausgebildete Abdichtelement 70 auf der Ausgabeseite des Magazins 26 unwirksam. Die Kapselung des vom Stößel 34 erfassten, aus dem Aufnahmeraum 40 herausgeschobenen Testelement 42 ist in diesem Zustand nicht mehr erforderlich, da der Benutzer das schieberförmige Griffstück 32 zum Ausschieben des Testelementes 42 an der Ausgabeöffnung 46 des tragbaren Analysegerätes 10 betätigt hat und somit die unmittelbar bevorstehende Benutzung des Testelementes 42 folgt.

Figur 4.4 ist die weitere Verdrehbewegung des Testelementes 42 ausgehend von der in Figur 3 dargestellten Position zu entnehmen.

Im Vergleich zur Anfangsphase der Verdrehbewegung gemäß Figur 4.3 ist das Testelement 42 gemäß der Darstellung in Figur 4.4 fast in seine Darreichungsposition 72 (vergleiche Figur 4.5) verdreht. Die Verdrehbewegung erfolgt nach Heranschieben des schieberförmig ausgebildeten Griffstückes 32 an die Längsseite des Magazins 26. Auch in der Darstellung gemäß Figur 4.4 ist das Magazin 26 entgegen der Wirkung des Vorspannelementes 28 in seiner Position innerhalb des tragbaren Analysegerätes 10 fixiert, da der erste Mitnehmerzahn 54 in die als Verzahnung ausgebildete Eingriffsstruktur 44 eingreift und eine laterale Bewegung des Magazins 26 in Vorschubrichtung 66 verhindert.

In vorteilhafter Weise ist die Teilung 60 an den beiden Längsseiten der Eingriffsstruktur 44 so ausgeführt, dass die Teilung 60, in welcher die Lücken zwischen den einzelnen Zähnen an beiden Längsseiten der Eingriffsstruktur 44 ausgeführt sind, dem Abstand zweier nebeneinander liegender Aufnahmeräume 40 des Magazins 26 entspricht. Auch in der Darstellung gemäß Figur 4.4 liegt der zweite Mitnehmerzahn 56 der Transportklinke 30 an einer Anschlagfläche 68 eines Zahnes auf der dem schlitzförmigen Griffstück 32 gegenüberliegenden Seite der Eingriffsstruktur 44 an.

Aus der Darstellung gemäß Figur 4.5 geht hervor, dass das Testelement 42 nach vollständiger Vorschubbewegung des Stößels 34 und nach Vollendung der Verdrehbewegung von seiner Speicherposition innerhalb des Magazin 26 in die in Figur 4.5 dargestellte Position eine 90°-Verdrehung erfahren hat und sich somit in seiner Darreichungsposition 72 befmdet.

Die Speicherposition der Testelemente 42 innerhalb des Magazins 26 ist dadurch charakterisiert, dass sich die einzelnen Testelemente 42 vor der Benutzung in luftdicht verschlossenen Hohlräumen 40 befinden. Die Testelemente 42 sind in ihrer Speicherposition im Wesentlichen in Hochkantlage innerhalb des Magazins 26 aufgenommen. In der Speicherposition der Testelemente 42 innerhalb des Magazins 26 können diese sowohl senkrecht zum Boden des Magazins 26 gespeichert als auch um einen Winkel geneigt zum Boden des Magazins 26 aufgenommen sein.

Die 90°-Verdrehung des Testelementes 42 ist in der Darstellung gemäß Figur 4.5 durch Bezugszeichen 72 identifiziert und entspricht der Darreichungsposition. Das den Stößel 34 betätigende, schieberförmig ausgebildete Griffstück 32 liegt in der in Figur 4.5 dargestellten Position an einer Längsseite des Magazins 26 an. Die Länge der den Stößel 34 partiell umgebenden Führung 36 ist vollständig durchfahren so dass in dieser Position das für den Benutzer zugängliche Ende des medizinischen Verbrauchsmaterials in Teststreifenform 42 aus der Ausgabeöffnung 46 am tragbaren Analysegerät 10 gemäß der Darstellung in Figur 2 ausgefahren ist. Der Figurensequenz der Figuren 4.1 bis 4.5 ist mithin zu entnehmen, dass die Verdrehbewegung des Testelementes 42 um eine Achse erfolgt, die parallel zur Ausschubbewegung der Testelemente 42 aus dem Magazin 26, 28 liegt. Die Verdrehbewegung von der Speicherposition der Testelemente 42 innerhalb des Magazins 26, 78 in die Darreichungsposition 72 kann demnach z. B. um die Längsachse der Testelemente 42 erfolgen, wie der Figurensequenz gemäß der Figuren 4.1 bis 4.5 zu entnehmen ist.

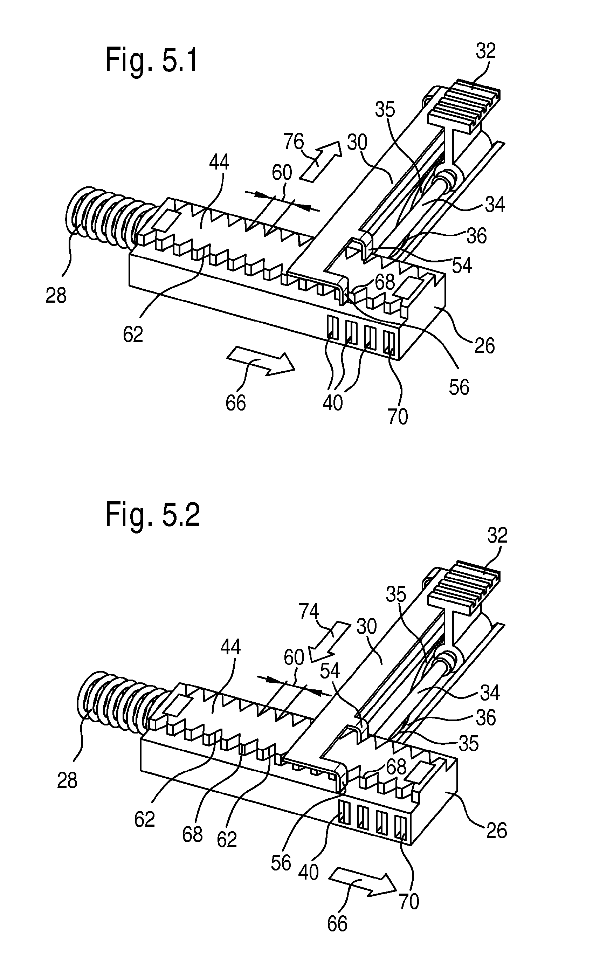

Den Darstellungen gemäß der Figuren 5.1 und 5.2 ist der Vorschub des Magazins 26 innerhalb des tragbaren Analysegerätes 10 zu entnehmen.

Aus der Darstellung gemäß Figur 5.1 geht hervor, dass aufgrund des in Figur 3 dargestellten Versatzes 58 des ersten Mitnehmerzahns 54 und des zweiten Mitnehmerzahns 56 an der Transportklinke 30, der zweite Mitnehmerzahn 56 in eine Zahnlücke der Eingriffsstruktur 44 eingreift, während der erste Mitnehmerzahn 54 in der Darstellung gemäß Figur 5.1 an einer mit Bezugszeichen 68 identifizierten Anschlagfläche 68 auf der gegenüberliegenden Längsseite der Eingriffsstruktur 44 anliegt. Die Bewegung der Transportklinke 30 erfolgt über eine Kopplung mit dem manuell betätigbaren Griffstück 32. Die Kopplung zwischen der Transportklinke 30 und dem Griffstück 32 kann z.B. über eine Federkopplung oder eine Einklinkmimik erfolgen, so dass die Transportklinke 30 nur während der Anfangsphase der Vorschubbewegung des Griffstücks 32 mit diesem gekoppelt ist und für eine Weiterschaltung des Magazins 26 in die durch mit Bezugszeichen 66 bezeichnete Vorschubrichtung des Magazins 26 sorgt. Die Weiterschaltung des Magazins 26, in dessen Hohlräumen 40 die Testelemente 42 aufgenommen sind, kann sowohl während der in Figur 5.1 mit Bezugszeichen 76 bezeichneten Bewegung der Transportklinke 30 als auch während der in Figur 5.2 durch Bezugszeichen 74 gekennzeichneten Hubbewegung der Transportklinke 30 erfolgen. Über die in die Ausnehmungen der Eingriffsstruktur 44 jeweils eingreifenden Mitnehmerzähne 54, 56 wird nach vorhergehendem Ausschub des Testelementes 42 aus den Hohlräumen 40 des Magazins 26 der in Vorschubrichtung 66 des Magazins 26 gesehene nächste Hohlraum 40 gegenüber dem mittels des Griffstücks 32 bewegbaren Stößels 34 gestellt, so dass dieser beim nächsten Anwendungszyklus, d.h. bei der nächsten Betätigung des Griffstücks 32 aus dessen Hohlraum 40 des Magazins 26 ausgeschoben werden kann.

Auf der die Eintrittseite des Stößels 34 darstellenden Längsseite des Magazins 26 können jeweils O-Ring-Dichtungen vorgesehen sein, welche auf eine ebenfalls auf der Eintrittsseite des Magazins 26 aufgebrachte Siegelfolie aufgebracht sein können. Fährt das der Eintrittsseite des Magazins 26 gegenüberliegende Ende des Stößels 34 in einen entsprechenden Hohlraum 40 ein, so wird die Siegelfolie bzw. eine Dichtlackschicht durchstoßen. Auf der Austrittsseite des Testelementes 42 kann ebenfalls eine Siegelfolie oder eine Dichtlackschicht aufgebracht sein. Diese wird durch den Vorschub des Testelementes 42 aus den Hohlräumen 40 ausgeschoben und durchstößt dabei auch auf dieser Seite die Dichtlackschicht bzw. die dort vorgesehene Siegelfolie. Weiterhin fungieren die auf der Austrittsseite in den Figuren 5.1 und 5.2 dargestellten elastischen Dichtelemente 70 in Form von Dichtlippen während der Ausschubbewegung des Testelementes 42 als Abdichtung während des Ausschiebens. Da im Magazin 26 die einzelnen Hohlräume 40 durch Trennwände voneinander getrennt sind, ist eine Kontamination der im Magazin 26 bevorrateten Testelemente 42 durch einen geöffneten Hohlraum 40 während der Ausschiebephase der Testelemente 42 aus diesem ausgeschlossen.

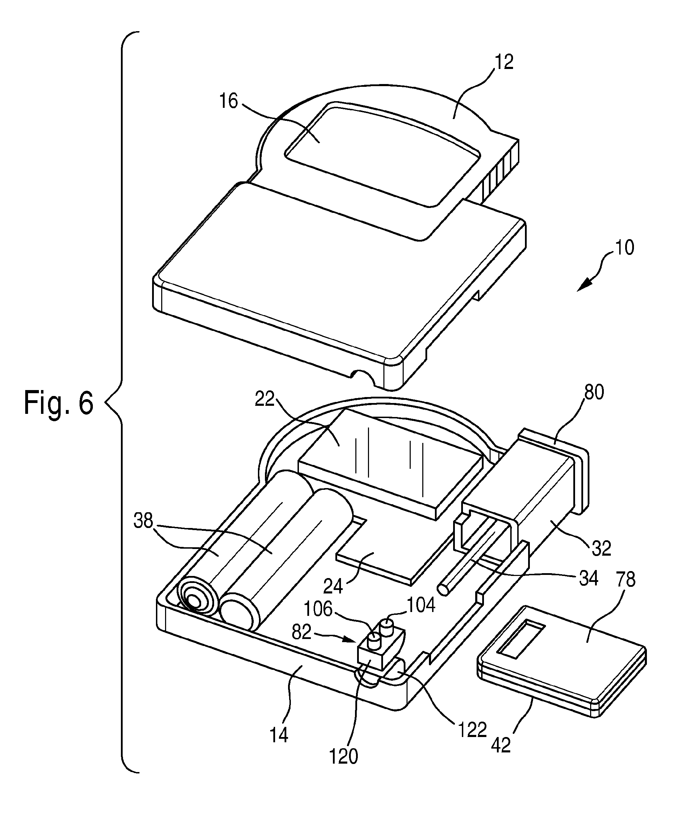

Figur 6 zeigt ein weiteres Ausführungsbeispiel des erfindungsgemäß vorgeschlagenen tragbaren Analysegerätes.

Aus der in Figur 6 dargestellten Explosionsdarstellung geht hervor, dass die Oberschale 12 eine Öffnung 16 aufweist. Die Öffnung 16 entspricht im Wesentlichen den Abmessungen eines auf der Platine 24 in der Unterschale 14 angeordneten Displays 22. Die Auswertelektronik des tragbaren Analysegerätes 10 in Figur 6 ist im Detail nicht dargestellt. Es sind die Batterien 38, die zur Stromversorgung des tragbaren Analysegerätes 10 dienen, wiedergegeben. Diese sind in eine Batterieaufnahme in der Unterschale 14 des tragbaren Analysegerätes 10 eingelassen. In der Unterschale 14 befindet sich darüber hinaus auch ein stempelförmig ausgebildetes Griffstück 32, welches eine Druckfläche 80 aufweist. Das stempelförmig ausgebildete Griffstück 32 umschließt einen stangenförmig ausgebildeten Stößel 34, der zum Ausschieben der innerhalb eines stationären Magazins 78 enthaltenen Testelemente 42 dient. Dem stangenförmigen Stößel 34 gegenüberliegend, befindet sich in der Unterschale 14 eine durch Bezugszeichen 82 identifizierte Verdreheinrichtung. Die Verdreheinrichtung 82 umfasst im Wesentlichen ein erstes Anschlagteil 120 und ein zweites Anschlagteil 122. Jedes der beiden Anschlagteile 120, 122 wird durch ein Paar von Anstellfedern 104, 106 beaufschlagt, so dass der sich zwischen den beiden Anschlagteilen 120, 122 einstellende Spalt variabel ist. Die am ersten Anschlagteil 120 dargestellten Federn 104 und 106 stützen sich an der Innenseite an der Oberschale 12 ab, während die in Figur 6 nicht dargestellten, das zweite Anschlagteil 122 jeweils beaufschlagenden Anstellfedern 104 und 106 an der Innenseite der Unterschale 14 des tragbaren Analysegerätes 10 abgestützt sind.

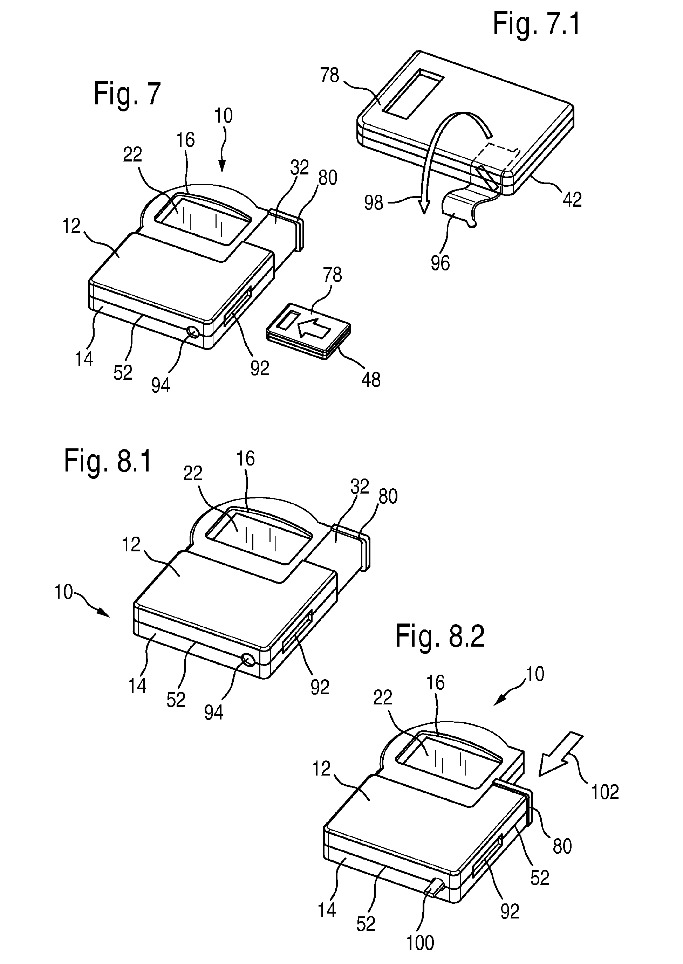

Der Darstellung in Figur 7 ist das tragbare Analysegerät 10 gemäß der Darstellung in Figur 6 in montiertem Zustand dargestellt. Durch die Öffnung 16 in der Oberschale 12 des tragbaren Analysegerätes 10 kann das Display 22 abgelesen werden. Die Oberschale 12 und die Unterschale 14 stoßen entlang einer Teilungsfuge 52 aneinander an. Entsprechend konfigurierte Ausnehmungen in der Oberschale 12 und in der Unterschale 14 definieren sowohl eine Ausgabeöffnung 94 für die Testelemente 42, die aus dem tragbaren Analysegerät 10 ausgeschoben werden, als auch die in rechteckförmigem Querschnitt ausgebildete Einschuböffnung 92 für das stationäre Magazin 78, welches in Einschubrichtung 48 in das tragbare Analysegerät 10 eingeschoben wird. Aus der Darstellung gemäß Figur 7 geht darüber hinaus hervor, dass sich das stempelförmig ausgebildete Griffstück 32 in seiner deaktivierten Position befindet, so dass der von diesem umschlossene Stößel 34 inaktiv ist. Mit Bezugszeichen 80 ist die manuell betätigbare Druckfläche des stempelförmig ausgebildeten Griffstücks 32 bezeichnet.

Der Darstellung gemäß Figur 7.1 ist das stationäre Magazin 78 in Form eines Stapelmagazins in vergrößerter Darstellung zu entnehmen. Neben der an der Oberseite des stationären Magazins 78 angeordneten Code-Key-Fläche zur Auslesung chargespezifischer Information durch die Auswertelektronik des tragbaren Analysegerätes 10, umfasst das stationäre Magazin 78 ein bewegbares Verschlusselement 96. Dieses kann in Richtung des Pfeils 98 geöffnet werden, so dass eine Ausgabeöffnung für das Testelement 42 aus dem stationären Magazin 78 geschaffen ist. Das Verschlusselement 96 kann als Kunststoffbauteil ausgeführt sein, welches im benutzten Zustand des stationären Magazins 78 den Eintritt von Luftfeuchtigkeit in das stationäre Magazin 78 verhindert, Nach Eröffnen des Verschlusselementes 96 hingegen, ist ein problemloses Ausschieben des jeweiligen Testelementes 42 aus dem stationären Magazin 78 möglich.

In Figur 8.1 ist das tragbare Analysegerät 10 im montierten Zustand dargestellt, wobei das stempelförmig ausgebildete Griffstück 32 samt Druckfläche 80 in seiner deaktivierten Position dargestellt sind, d.h. in diesem Zustand des Analysegerätes 10 wird an der Ausgabeöffnung 94 des tragbaren Analysegerätes 10 kein Testelement 42 dargeboten. In der Darstellung gemäß Figur 8.2 ist das stempelförmig ausgebildete Griffstück 32 im in das tragbare Analysegerät 10 eingefahrenen Zustand dargestellt. Dieser Zustand wird durch Drücken auf die Druckfläche 80 erreicht, wodurch eine Kraft 102 auf das stempelförmig ausgebildete Griffstück 32 wirkt, so dass dieses in das die Oberschale 12 und die Unterschale 14 umfassende Gehäuse des tragbaren Analysegerätes 10 einfährt. Aus dem in die Einschuböffnung 92 eingeschobenen stationären Magazin 78 wird das jeweils nächstliegende, durch den verfahrbaren Stößel 34 ausschiebbare Testelement 42 an der Ausgabeöffnung 94 dargeboten, in Figur 8.2 dargestellt durch Bezugszeichen 100. Der Vollständigkeit halber sei erwähnt, dass die Oberschale 12 und die Unterschale 14 entlang der Teilungsfuge 52 aneinander liegen. Die Teilungsfuge kann z.B. auch als Labyrinthdichtung ausgebildet sein, um den Eintritt von Luftfeuchtigkeit in den Innenraum des tragbaren Analysegerätes 10 soweit wie möglich zu minimieren.

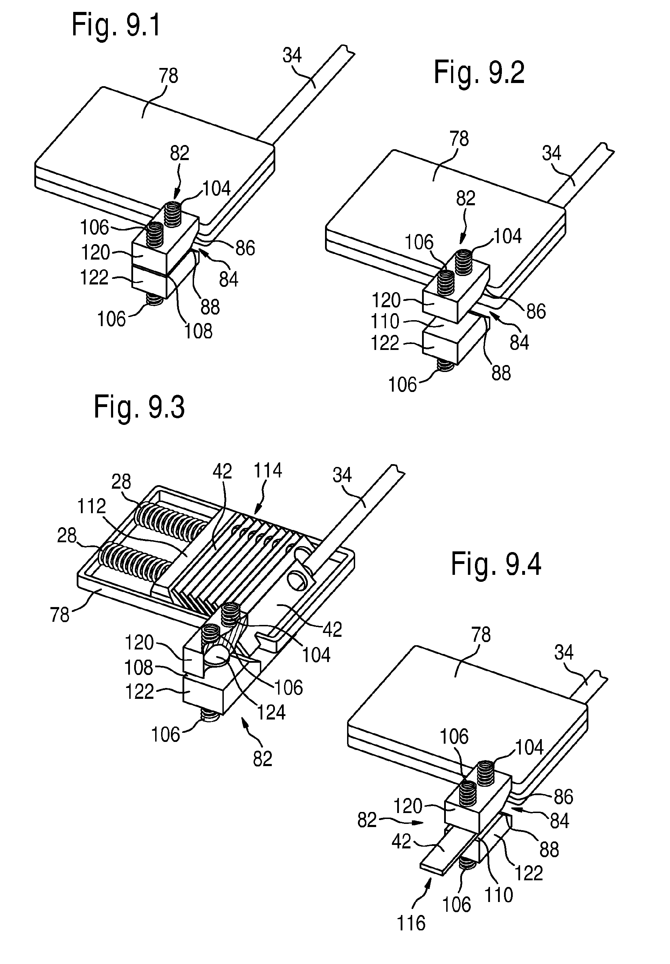

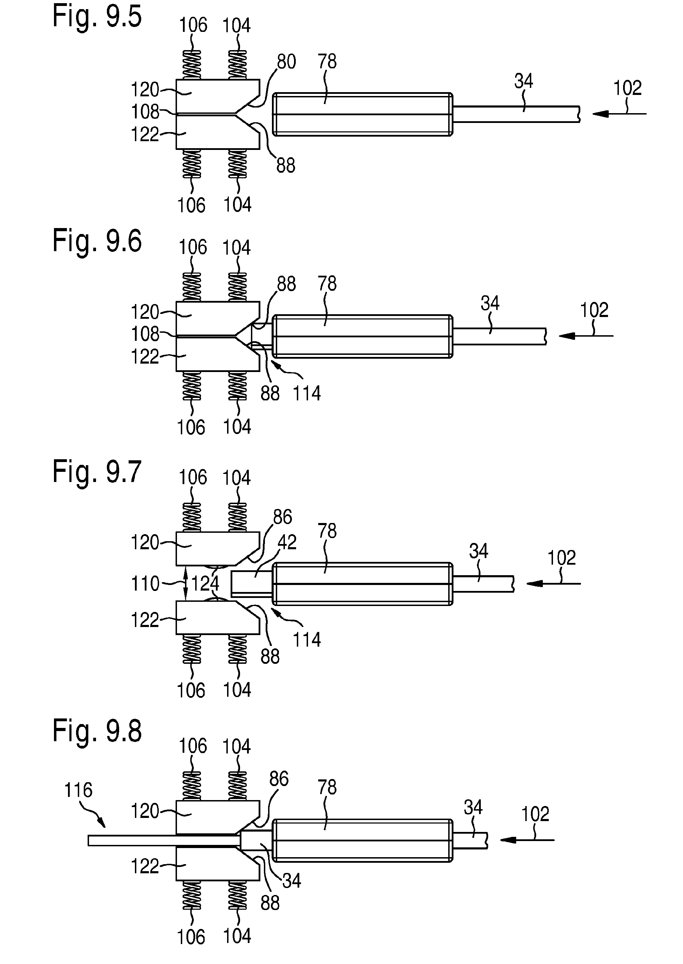

Der Darstellung gemäß Figur 9.1 ist eine dem stationären Magazin 78 gegenüberliegend angeordnete Verdreheinrichtung 82 entnehmbar. Gemäß der Darstellung in Figur 9.1 umfasst die Verdreheinrichtung 82 das erste Anschlagteil 120 sowie das zweite Anschlagteil 122. Die beiden einander gegenüberliegenden Seiten des ersten Anschlagteils 120 sowie des zweiten Anschlagteiles 122 begrenzen einen Spalt, der in der Darstellung gemäß Figur 9.1 eine enge Spaltweite 108 aufweist. Jedes der Anschlagteile 120 bzw. 122 ist durch ein Paar von Anstellfedern 104, 106 beaufschlagt, wodurch die beiden Anschlagteile 120, 122 relativ zueinander bewegbar sind. Bei in das tragbare Analysegerät 10 eingeschobenem stationärem Magazin 78, liegt dessen Ausgabeseite, d.h. die Ausschubseite des Testelementes 42 der Verdreheinrichtung 82 gegenüber. Der Stößel 34 liegt mit seiner Stirnseite hingegen der der Ausgabeseite des stationären Magazins 78 gegenüberliegenden Längsseite gegenüber. Sowohl das erste Anschlagteil 120 als auch das zweite Anschlagteil 122 umfassen jeweils Einlaufschrägen 86 bzw. 88, die einen Einlauftrichter 84 definieren.

Der Figur 9.2 ist entnehmbar, dass der Stößel 34 zum Ausschub des Testelementes 42 teilweise in das stationäre Magazin 78 eingeschoben ist. Aufgrund dessen wird das Testelement 42 in einer der Speicherposition im stationären Magazin 78 entsprechenden Orientierung aus diesem ausgeschoben. Bevorzugt sind die Testelement 42 innerhalb des stationären Magazins 78 in einer Schräglage z.B. um 45° geneigt im Bezug auf die Bodenfläche des stationären Magazins 78, aufgenommen. Aufgrund dieses Umstandes fährt das jeweils durch den Stößel 34 erfasste Testelement 42 in dieser Schräglage aus dem stationären Magazin 78 aus. Das Testelement 42 stößt and den Einlaufschrägen 86 bzw. 88 der durch die Anstellfedern 104, 106 beaufschlagten Anschlagteile 120 bzw. 122 an und vergrößert den Spalt zwischen diesen von der engen Spaltweite 108 auf die in Figur 9.2 dargestellte weite Spaltweite 110.

In Figur 9.3 ist eine Innenansicht des stationären Magazins 78 zu dargestellt. Aus der Darstellung gemäß Figur 9.3 geht einerseits die 45°-Orientierung 114 (Speicherposition) des Testelementes 42 innerhalb des stationären Magazins 78 hervor, sowie andererseits die Fördermimik des Testelementes 42 innerhalb des stationären Magazins 78. Innerhalb des stationären Magazins 78 sind gemäß der Darstellung in Figur 9.3 Vorspannelemente 28 angeordnet, die z.B. als Spiralfedern ausgebildet sein können. Ein Paar von Spiralfedern 28 beaufschlagt einen Anschlag 112, dessen von den Vorspannelementen 28 abgewandte Seite dem Vorrat von Testelementen 42 zuweist. Diese sind demnach stets vorgespannt innerhalb des stationären Magazins 78 angeordnet, so dass sicher gestellt ist, dass bei Einfahrbewegung des Stößels 34 in das stationäre Magazin 78, stets ein neues und Testelement 42 aus diesem ausgeschoben werden kann und in die Verdreheinrichtung 82 eintritt.

Der Darstellung gemäß Figur 9.3 ist das erste Anschlagteil 120 in teilweise geschnittener Darstellung zu entnehmen. Das durch die Anstellfedern 104 und 106 beaufschlagte erste Anschlagteil 120 umfasst eine Rundung 124, auf welche das vorlaufende Ende des aus dem stationären Magazin 78 ausgeschobenen Testelements 42 aufläuft. Durch die Vorschubbewegung des Stößels 34 wird eine Kraft auf das Testelement 42 ausgeübt. Das auf die Einlaufschrägen 86 bzw. 88 auflaufende Testelement 42 weitet bei weiterem Einschieben in den durch das erste Anschlagteil 120 und das zweite Anschlagteil 122 begrenzten engen Spalt 108 diesen bis auf die in Figur 9.2 dargestellte weite Spaltweite 110 auf und passiert dabei die Verrundungen 124, die am ersten Anschlagteil 120 bzw. am zweiten Anschlagteil 122 ausgebildet sind um einen schonenden Transport des schräg stehenden, vom Stößel 34 ergriffenen Testelements 42 durch die Verdreheinrichtung 82 zu gewährleisten.

Der Darstellung gemäß Figur 9.4 ist entnehmbar, dass nach vollständigem Ausschieben des Testelements 42 aus dem stationären Magazin 78 durch den Stößel 34 dieser in eine horizontale Orientierung 116 überführt ist. Sobald das Testelement 42 durch die Stirnseite des Stößels 34 aus dem stationären Magazin 78 ausgeschoben ist, wird dieser von seiner Speicherlage, welche der in Figur 9.3 dargestellten 45°-Orientierung 114 entspricht, in die horizontale Orientierung 116 überführt. Dies resultiert aus der Beaufschlagung sowohl des ersten Anschlagteiles 120 als auch des zweiten Anschlagteils 122 durch die Anstellfedern 104, 106. Die in Figur 9.2 dargestellte aufgeweitete Spaltweite 110 wird in die enge Spaltweite 108 überführt, die durch die Dicke des Testelements 42 definiert ist. In dem in Figur 9.4 dargestellten Zustand beaufschlagen die Verrundungen 124 die Ober- bzw. die Unterseite des Testelements 42, so dass dessen materialschonende Fixierung durch die Verdreheinrichtung 82 gewährleistet ist.

Der Darstellung der Figurensequenz der Figuren 9.5. bis 9.8 sind die Transportphasen des Testelementes 42 aus dem stationären Magazin 78 und der Vorschub des Testelementes 42 durch die Verdreheinrichtung 82 zu entnehmen.

In der Darstellung gemäß Figur 9.5 sind das erste Anschlagteil 120 und das zweite Anschlagteil 122, jeweils beaufschlagt durch ein Paar von Anstellfedern 104 und 106, aneinander angestellt, d.h. es liegt ein enger Spalt 108 vor. Der Stößel 34 ist in diesem Zusammenhang noch nicht in das stationäre Magazin 78, welches bevorzugt ein Stapelmagazin ist, hineingeschoben. Der Einlauftrichter 84 wird durch die erste Einlaufschräge 86 und die zweite Einlaufschräge 88 am ersten Anschlagteil 120 bzw. am zweiten Anschlagteil 122 definiert.

Figur 9.6 ist entnehmbar, dass der Stößel 34 teilweise in das stationäre Magazin 78 eingeschoben ist. Die Stirnseite des Stößels 34 schiebt ein in 45°-Orientierung 114 (Speicherposition) im stationären Magazin 78 aufgenommenes Testelement 42 aus diesem hinaus. In der Darstellung gemäß Figur 9.6 kontaktieren die Kanten des vorlaufenden Endes des Testelementes 42 die Einlaufschrägen 86 bzw. 88 des ersten Anschlagteiles 120 bzw. des zweiten Anschlagteiles 122. Es besteht nach wie vor der enge Spalt 108 zwischen den einander zuweisenden Seiten des ersten Anschlagteils 120 sowie des zweiten Anschlagteils 122.

Figur 9.7 ist ein weiterer Vorschub des Stößels 34 und demnach eine weitere Förderung des vorlaufenden Endes des Testelementes 42 zu entnehmen. Durch das Passieren des Einlauftrichters 84, das durch die erste Einlaufschräge 86 und die zweite Einlaufschräge 88 begrenzt ist, fahren das erste Anschlagteil 120 und das zweite Anschlagteil 122 gegen die Wirkung der Anstellfedern 104 und 106 auf. Das sich noch in seiner Speicherposition 114 im stationären Magazin 78 befmdende, vom Stößel 34 erfasste Testelement 42 wird nun zwischen die vorgespannten Anschlagteile 120 bzw. 122 der Verdreheinrichtung 82 geschoben. Figur 9.8 ist entnehmbar, dass das Testelement 42 nunmehr vollständig in seiner ganzen Länge aus dem stationären Magazin 78 ausgeschoben ist, welches nunmehr vollständig vom Stößel 34 durchsetzt ist. Aufgrund der Federbeaufschlagung des ersten Anschlagteiles 120 sowie des zweiten Anschlagteiles 122 durch die Anstellfedern 104, 106, wird das Testelement 42 von seiner 45°-Orientierung 114 (Speicherposition) in eine horizontalen Position 116 (Darreichungsposition) gemäß der Darstellung in Figur 9.8 verdreht. In der Darreichungsposition 116 bzw. 100 (vgl. Figur 8.2) ist das Testelement 42 durch den Benutzer des tragbaren Analysegerätes 10 benutzbar, wie in Figur 8.2 dargestellt.

Der Figurensequenz der Figuren 9.5 bis 9.8 ist zu entnehmen, dass die Testelemente 42 eine Verdrehbewegung um eine Achse parallel zur Vorschubrichtung der Testelemente 42 erfahren. Bei dieser Achse handelt es sich bevorzugt um die Längsachse der Testelemente 42. Die Einleitung der Verdrehbewegung in die Testelemente erfolgt durch die Anschlagteile 120 bzw. 122 bzw. deren Einlaufschrägen 86 und 88. Die Anschlagteile 120 bzw. 122 sind Komponenten der Verdreheinrichtung 82. Auch mit der im Zusammenhang mit der vorstehenden Figurensequenz erwähnten Verdreheinrichtung 82 werden die Testelemente 42 von ihrer Speicherposition innerhalb des Magazins 78 in eine Darreichungsposition 116 überführt.

Mit den beiden Ausführungsbeispielen des der Erfindung zugrunde liegenden Gedankens wird erreicht, dass in einem stationären Magazin 78 aufgenommene Testelemente 42 gegen eindringende Luftfeuchtigkeit geschützt ist, wobei innerhalb des stationären Magazins 78 eine sehr hohe Packungsdichte der Testelemente 42 erreicht werden kann, so dass eine mehreren Tagen entsprechende Dosis von Testelementen 42 zur Benutzung durch einen Patienten in ein tragbares Analysegerät 10 eingebracht werden kann, ohne dass die Testelemente 42 durch Luftfeuchtigkeit kontaminiert würden und damit unbrauchbar wären. Aufgrund der dargestellten, sich an den Ausschiebevorgang aus dem stationären Magazin 78 anschließenden Verdrehbewegung der Testelemente 42 kann dieses in einer für Benutzer des tragbaren Analysegerätes 10 bequemen Weise benutzt werden.

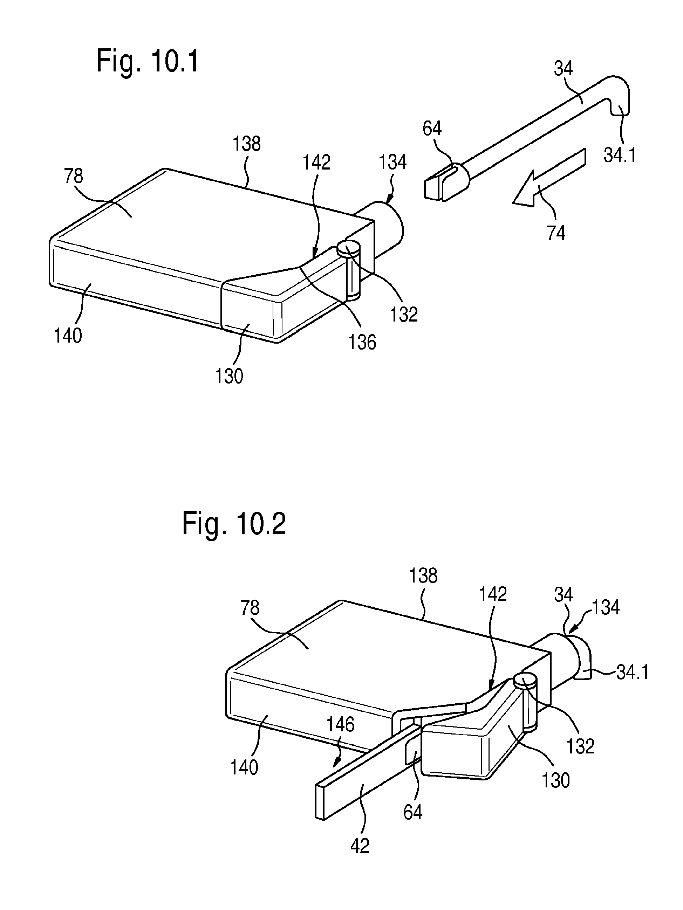

Figuren 10.1 und 10.2 zeigen ein klappenförmig ausgeführtes Dichtelement für ein stationär im Gehäuse eines tragbaren Analysegeräts oder eines tragbaren Analysesystems aufgenommenes Magazin.

Figur 10.1 zeigt das stationäre Magazin 78, dessen Eintritts-Längsseite für den Stößel 34 durch Bezugszeichen 138 und dessen Ausschub-Längsseite mit Bezugszeichen 140 identifiziert ist. Am stationären Magazin 78 ist eine um ein Gelenk 132 schwenkbare Dichtklappe 130 angeordnet, die sich in der Darstellung gemäß Figur 10.1 in ihrer Dichtposition 142 befmdet. Der Dichtspalt zwischen dem stationären Magazin 78 und der Dichtklappe 130 ist durch Bezugszeichen 136 identifiziert. Der Stößel 64 wird an der Eintritt-Längsseite 138 des stationären Magazins 78 in eine Einführöffnung 134 eingeschoben, in der sich ein vorzugsweise ringförmig ausgebildetes Dichtelement befindet. Im Inneren des stationären Magazins 78 gemäß der Darstellung in Figur 10.1 ist das (vgl. Darstellung gemäß Figur 9.3) Testelement 42 in Schrägstellung 114 (Speicherposition) aufgenommen.

Wird der Stößel 34 in Vorwärtshubrichtung 74 in die Einführöffnungen 134 eingefahren, so wird aufgrund einer Kopplung des Gelenks 132 für die Dichtklappe 130 nach beispielsweise dem Kulissenprinzip die Dichtklappe 130 entsprechend der Vorwärtshubbewegung 74 des Stößels 34 per Griffstück 32 geöffnet.

In der Darstellung gemäß Figur 10.2 befindet sich die Dichtklappe im stationären Magazin in ihrer Freigabeposition. Gemäß der Darstellung in Figur 10.2 ist der Stößel 34 vollständig in das stationäre Magazin 78 eingefahren und hat das Testelement 42 innerhalb der Schlitzung 64 ergriffen und in eine Ausschubposition 146 bewegt. Die sich nun in ihrer Ausgabeposition 144 befindende Dichtklappe 130 ist nur so weit geöffnet, dass das Testelement 42 aus diesem herauszutreten vermag. Aufgrund der Kopplung der Dichtklappe 130 mit der Hubbewegung des Stößels 34 wird bei einer Ausfahrbewegung des Stößels 34 aus dem stationären Magazin 78 durch dementsprechende Betätigung des Griffstücks 32 die Dichtklappe wieder in ihre in Figur 10.1 dargestellte Dichtposition 142 gestellt.

Innerhalb der Einführöffnung 134 kann eine Langzeitabdichtung des stationären Magazins 78 gegen eintretende Luftfeuchtigkeit dadurch erreicht werden, dass neben einer O-ringförmig ausgebildeten Dichtlippe innerhalb der Einführöffnung 134 an einem von der Einführöffnung 134 überdeckten Bereich der Eintritt-Längsseite 138 ein Dichtlack oder eine Siegelfolie aufgebracht sein kann, welche beim erstmaligen benutzen des stationären Magazins 78 durch Betätigen des Stößels 34 von dessen Schlitzung 64 durchstoßen werden kann.

### Bezugszeichenliste

- 10: Tragbares Analysegerät/Analysesystem
- 12: Oberschale
- 14: Unterschale
- 16: Öffnung
- 18: schlitzförmige Öffnung
- 20: Verschlussklappe
- 22: Display
- 24: Platine
- 26: Magazin (Stapelmagazin)
- 28: Vorspannelement
- 30: Transportklinke für Magazin 26
- 32: schieberförmiges Griffstück/stempelförmiges Griffstück
- 34: Stößel
- 34.1: abgewinkeltes Ende Stößel
- 35: Kurvenabschnitt
- 36: Führung
- 38: Energiespeicher
- 40: Aufnahmeraum
- 42: Testelement
- 44: Eingriffsstruktur
- 46: Ausgabeöffnung
- 48: Einschubrichtung Magazin 26
- 50: Einschuböffnung
- 52: Teilungsfuge
- 54: Erster Mitnehmerzahn
- 56: Zweiter Mitnehmerzahn
- 58: Versatz Mitnehmerzähne 54, 56
- 60: Teilung
- 62: Einlaufschräge
- 64: Schlitzung
- 66: Vorschubrichtung Magazin 26
- 68: Anlagefläche von Eingriffsstruktur 44
- 70: Abdichtelement
- 72: Darreichungsposition Testelement
- 74: Vorwärtshub
- 76: Rückwärtshub

- 78: stationäres Magazin
- 80: Druckfläche
- 82: Verdreheinrichtung
- 84: Einlauftrichter
- 86: erste Einlaufschräge
- 88: zweite Einlaufschräge
- 92: Einschuböffnung für Magazin 26
- 94: Ausgabeöffnung für Testelement 42
- 96: Verschlusselement
- 98: Öffnungsrichtung
- 100: Darreichungsposition Testelement 42
- 102: Betätigungskraft
- 104: erste Anstellfeder
- 106: zweite Anstellfeder
- 108: enger Spalt
- 110: aufgeweiteter Spalt
- 112: federnder Anschlag
- 114: 45°-Orientierung
- 116: Darreichungsposition
- 120: erstes Anschlagteil
- 122: zweites Anschlagteil
- 124: Verrundung

- 130: Dichtklappe
- 132: Gelenk mit Kulissenkopplung
- 134: Einführöffnung
- 136: Dichtspalt
- 138: Eintritts-Längsseite
- 140: Ausschub-Längsseite
- 142: Dichtposition Dichtklappe
- 144: Ausgabeposition Dichtklappe
- 146: Ausschubposition Testelement 42

## Patentansprüche

1. Tragbares Analysegerät (10) mit einem von einem Gehäuse (12, 14) begrenzten Innenraum und mit einem Magazin (26, 78) zur Aufnahme von Testelementen (42), mit einer Fördereinrichtung (32, 34, 36) zum Fördern der Testelemente (42) von einer ersten Position im Inneren des Magazins (26, 78) in eine zweite zumindest teilweise außerhalb des Magazins (26, 78) und innerhalb des Gehäuses (12, 14) liegende Position, wobei das Fördern der Testelemente (42) aus dem Magazin (26, 78) in zwei Bewegungen erfolgt, wobei das Fördern der Testelemente (42) aus dem Magazin (26, 78), in dem diese in einer Speicherposition (110, 114) enthalten sind, derart erfolgt, dass die Testelemente (42) mittels einer Verdreheinrichtung (35, 36; 82) in eine Darreichungsposition (72, 100, 116) bewegt werden.

2. Tragbares Analysegerät gemäß Anspruch 1, **dadurch gekennzeichnet, dass** die Speicherposition (110, 114) der Testelemente (42) innerhalb des Magazins (26, 78) durch deren Hochkantlage im Magazin (26) gegeben ist.

3. Tragbares Analysegerät gemäß Anspruch 1, **dadurch gekennzeichnet, dass** die Speicherposition (110, 114) der Testelemente (42) innerhalb des Magazins (78) durch deren Schrägstellung (114) innerhalb des Magazins (78) gegeben ist, welches im Gehäuse (12, 14) ortsfest positioniert ist.

4. Tragbares Analysegerät gemäß Anspruch 1, **dadurch gekennzeichnet, dass** die Darreichungsposition (72, 116) Testelemente (42) durch deren Horizontallage an einer Ausgabeöffnung (46, 94) des Gehäuses (12, 14) definiert ist.

5. Tragbares Analysegerät gemäß der Ansprüche 1, 2 oder 3, **dadurch gekennzeichnet, dass** die Bewegung Testelemente (42) von deren Speicherposition (110, 114) innerhalb des Magazins (26, 78) in die Darreichungsposition (72, 100, 116) eine Drehbewegung ist.

6. Tragbares Analysegerät gemäß Anspruch 1, **dadurch gekennzeichnet, dass** die Fördereinrichtung (32, 34, 36) einen mittels eines Griffstückes (32) manuell betätigbaren Stößel (34) aufweist, bei dessen Vorschubbewegung (74) relativ zum innerhalb des Gehäuses (12, 14) arretierten Magazins (26), ein Testelement (42) aus diesem ausgeschoben wird.

7. Tragbares Analysegerät gemäß Anspruch 1, **dadurch gekennzeichnet, dass** das Magazin (26) eine Eingriffsstruktur (44) aufweist, in welche eine Transportklinke (30) zum Vorschub des Magazins (26) in Vorschubrichtung (64) innerhalb des Gehäuses (12, 14) eingreift.

8. Tragbares Analysegerät gemäß Anspruch 7, **dadurch gekennzeichnet, dass** die Transportklinke (30) Mitnehmer (54, 56) aufweist, die in einem Versatz (58) relativ zueinander angeordnet sind.

9. Tragbares Analysegerät gemäß Anspruch 7, **dadurch gekennzeichnet, dass** die Eingriffsstruktur (44) am Magazin (26) parallel zu dessen Vorschubrichtung (64) verläuft und als durch Einlaufschrägen (62) und Anlageflächen (68) dargestellte Zahnreihenstruktur ausgeführt ist.

10. Tragbares Analysegerät gemäß Anspruch 9, **dadurch gekennzeichnet, dass** die Einlaufschrägen (62) und die Anschlagflächen (68) an beiden Längsseiten der Eingriffsstruktur (44) ausgeführt sind.

11. Tragbares Analysegerät gemäß Anspruch 6, **dadurch gekennzeichnet, dass** der Stößel (34) in einer Führung (36) axial geführt ist, die dem Stößel (34) eine Verdrehbewegung aufprägt.

12. Tragbares Analysegerät gemäß Anspruch 11, **dadurch gekennzeichnet, dass** die Führung (36) den Stößel (34) in Umfangsrichtung zumindest teilweise umschließt und ein Kurvenabschnitt (35) aufweist, welcher den Stößel (34) nach vollständigem Ausschub Testelemente (42) eine Verdrehbewegung aufprägt.

13. Tragbares Analysegerät gemäß Anspruch 6, **dadurch gekennzeichnet, dass** der Stößel (34) an seinem dem Magazin (26) abgewandten Ende ein abgewinkeltes Ende (34.1) aufweist.

14. Tragbares Analysegerät gemäß Anspruch 6, **dadurch gekennzeichnet, dass** der Stößel (34) an seiner dem Magazin (26) zuweisenden Stirnseite eine Aufnahmeöffnung (64) zur Erfassung der Testelemente (42) aufweist.

15. Tragbares Analysegerät gemäß Anspruch 14, **dadurch gekennzeichnet, dass** die Aufnahmeöffnung (64) als Schlitzung im Stößel (34) ausgeführt ist.

16. Tragbares Analysegerät gemäß Anspruch 11, **dadurch gekennzeichnet, dass** die Verdrehbewegung der Testelemente (42) von der Speicherposition (110, 114) in Aufnahmeräumen (40) des Magazins (26) um 90° in die Darreichungsposition (72, 100, 116) an der Ausgabeöffnung (46, 94) verläuft.

17. Tragbares Analysegerät gemäß Anspruch 7, **dadurch gekennzeichnet, dass** die Transportklinke (30) mit dem Griffstück (32) zumindest während der Anfangsphase von dessen Vorwärtshub (74) gekoppelt ist.

18. Tragbares Analysegerät gemäß der Ansprüche 2 oder 3, **dadurch gekennzeichnet, dass** die Testelemente (42) aufnehmende Magazin (26) elastische Dichtelemente (70) aufweist, die den Innenraum des Magazins (26) sowohl im ungebrauchten Zustand des Magazins (26) als auch bei Entnahme eines Testelementes (42) gegen eintretende Luftfeuchtigkeit abdichten.

19. Tragbares Analysegerät gemäß Anspruch 18, **dadurch gekennzeichnet, dass** die elastischen Dichtelemente (70) als Dichtlippen ausgebildet sind, welche Aufnahmeräume (40) für die Testelemente (42) im Magazin (26) abdichten und bei Ausschub desselben aus dem Magazin (26) dichtend anliegen.

20. Tragbares Analysegerät gemäß Anspruch 3, **dadurch gekennzeichnet, dass** Testelemente (42) im Magazin (78) federbeaufschlagt aufgenommen sind.

21. Tragbares Analysegerät gemäß Anspruch 3, **dadurch gekennzeichnet, dass** die innerhalb des Gehäuses (12, 14) angeordnete Verdreheinrichtung (82) einander gegenüberliegende, mindestens eine Spaltweite (108, 110) definierende Körper (120, 122) umfasst.

22. Tragbares Analysegerät gemäß Anspruch 21, **dadurch gekennzeichnet, dass** mindestens einer der Körper (120,122) im Gehäuse (12, 14) federnd gelagert ist,

23. Tragbares Analysegerät gemäß Anspruch 21, **dadurch gekennzeichnet, dass** mindestens einer der als Führungsflächen fungierenden Körper (120, 122) eine Einlaufschräge (86,88) aufweist.

24. Tragbares Analysegerät gemäß Anspruch 21, **dadurch gekennzeichnet, dass** die die mindestens eine Spaltweite (108, 110) definierenden Körper (120, 122) einen Einlauftrichter (84) für die Testelemente (42) definieren.

25. Tragbares Analysegerät gemäß Anspruch 21, **dadurch gekennzeichnet, dass** zumindest eine der beiden einander zuweisenden Seiten der als Führungsflächen für die Testelemente (42) dienenden Körper (120,122) eine Verrundung (124) aufweist.

26. Tragbares Analysegerät gemäß der Ansprüche 2 oder 3, **dadurch gekennzeichnet, dass** das Magazin (26, 78) an zumindest einer seiner Längsseiten (138, 140) mit einer Siegelfolie oder einer Dichtlackschicht gegen das Eintreten von Luftfeuchtigkeit abgedichtet ist.
